# EUROPÄISCHE PATENTSCHRIFT

(11) **EP 0 548 088 B1**
(45) Veröffentlichungstag und Bekanntmachung des Hinweises auf die Patenterteilung: **14.01.1998**
(21) Anmeldenummer: 91912939.5
(22) Anmeldetag: 16.07.1991
(51) Int. Cl.: A61K 47/00, A61K 47/26, A61K 47/14

(54) **VERFAHREN ZUR HERSTELLUNG VON MIKRONISIERTE WIRKSTOFFE ENTHALTENDE HALBFESTE ZUBEREITUNGEN**
METHOD OF PRODUCING SEMI-SOLID PREPARATIONS CONTAINING MICRO-PARTICULATE SUBSTANCES
PROCEDE DE FABRICATION DE PREPARATIONS SEMI-SOLIDES RENFERMANT UNE MATIERE ACTIVE MICRONISEE

(43) Veröffentlichungstag der Anmeldung: 30.06.1993
(60) Teilanmeldung: 96250089.8
(73) Patentinhaber: SCHERING AKTIENGESELLSCHAFT, 13342 Berlin (DE)
(72) Erfinder: STINDL, Wolfgang, A-7000 Eisenstadt (AT); LEITNER, Erich, Dr., A-1090 Wien (AT); TACK, Johannes, Dr., D-1000 Berlin 20 (DE); DARGEL, Erwin, Dr., D-1000 Berlin 10 (DE)
(86) Internationale Anmeldenummer: DE9100593
(87) Internationale Veröffentlichungsnummer: WO9301836

(56) Entgegenhaltungen:
- DE-A- 4 012 514
- FR-A- 2 207 692

## Beschreibung

Die Erfindung betrifft ein Verfahren zur Herstellung von mikronisierte Wirkstoffe enthaltende halbfeste Zubereitungen, dadurch gekennzeichnet, daß man ein Gemisch aus einem oder zwei nichtionischen Tensiden mit der 2,3 bis 100-fachen Gewichtsmenge eines Fettsäureesters der allgemeinen Formel

R₁-COO-R₂

worin
R₁ und R₂ jeweils Alkylgruppen oder Alkenylgruppen mit 12 bis 32 Kohlenstoffatomen darstellen, oder einem Gemisch dieser Fettsäureester erwärmt bis es schmilzt, die Schmelze unter Rühren in die 10 bis 200-fache Gewichtsmenge einer halbfesten Zubereitung, die etwa auf die gleiche Temperatur erwärmt ist, einträgt, die Mischung unter Rühren erkalten läßt und mit dem mikronisierten Wirkstoff oder Wirkstoffgemisch sowie gewünschtenfalls mit Duftstoffen versetzt, wobei der mikronisierte Wirkstoff oder das Wirkstoffgemisch eine Korngröße von maximal 80 µm hat, und wobei das Gemisch aus zwei Tensiden einen HLB-Wert von 9 bis 18 hat.

Die nach dem erfindungsgemäßen Verfahren hergestellten halbfesten Zubereitungen ermöglichen eine bessere Verfügbarkeit des Wirkstoffs oder Wirkstoffgemisches als die in konventioneller Weise hergestellten, mikronisierte Wirkstoffe enthaltenden halbfesten Formulierungen. Dies hat zur Folge, daß die erfindungsgemäßen Zubereitungen wesentlich geringere Wirkstoffkonzentrationen benötigen um die gleiche Wiksamkeit zu entfalten, wie die auf konventionellem Wege hergestellten Formulierungen.

Das eigentliche, die Verfügbarkeit fördernde Substanzgemisch (nachfolgend als "Substanzgemisch" bezeichnet) ist eine Mischung aus ein oder zwei nichtionischen Tensiden mit einem HLB-Wert von 9 bis 18 und der 2,3 bis 100-fachen Gewichtsmenge (insbesondere der 2,4 bis 30 fachen Gewichtsmenge) eines Fettsäureesters der allgmeinen Formel

R₁-COO-R₂

worin
R₁ und R₂ gleich oder verschieden sind und jeweils eine Alkylgruppe oder eine Alkenylgruppe mit 12 bis 32 Kohlenstoffatomen darstellen oder einem Gemisch dieser Fettsäureester.

Nichtionische Tenside, die sich zur Herstellung des "Substanzgemisches" eignen sind beispielsweise in Ullmanns Enzyklopädie der technischen Chemie 4. Auflage, Band 22 (Verlag Chemie; DE-Weinheim et. al., 1982, Seite 488-496 und 1983 in "International Mc Cutcheon's Emulsifiers Detergents/Functional Materials" (Glen Rock NJ07452; USA) beschrieben.

Geeignete Tenside sind insbesondere in Wasser lösliche und in Paraffinöl unlösliche Oxethylate und innerhalb dieser vorzugsweise Oxethylate die endständig blockiert sind. Solche Tenside sind beispielsweise
Polysorbat 60 (Hersteller: ICI)
PEG-2000-stearat (Hersteller: ICI)
PEG-400-stearat (Hersteller: BASF)
Pluronic® P103 (Products Chimiques Ugine-Kuhlmann)
Arlatone®T (Atlas Chemical Industries N.V.)
Tween®21 (Atlas Chemical Industries N.V.) und
Myrj®51 (Atlas Chemical Industries N.V.)

Verwendet man zur Herstellung des "Substanzgemisch" eine Mischung aus 2 Tensiden, so muß diese Mischung einen HLB-Wert von 9 bis 18 haben, die Einzelkomponenten können aber einen niedrigeren oder auch höheren HLB-Wert besitzen. Geeignete Tensidgemische sind beispielsweise solche, die neben den obengenannten Oxethylaten noch Fettsäureester von Polyhydroxyverbindungen (wie Glycerin, Sorbit, Saccharose oder Pentaerythrit) enthalten.

Ein Fettsäureester ist beispielsweise das Pentaerythritmonostearat.

Fettsäureester der bereits erwähnten Formel

R₁-COO-R₂,

die sich zur Herstellung des "Substanzgemisch" eignen sind vorzugsweise solche, die insgesamt 28 bis 58 Kohlenstoffatome und insbesondere solche die 32 bis 48 Kohlenstoffatome besitzen.

Solche Fettsäureester sind beispielsweise der Octadecansäure-octadecenylester, der Hexadecansäure-eicosylester, der Octadecansäure-octadecylester, der Eicosensäure-docosenylester, der 13-Docosensäureeicosylester, der 9-Octadecensäure-tetracosylester, der 12-Triacontensäure-dodecylester, der Eicosansäure-docosylester, der Octadecansäure-tetracosylester und der 10-Octacosensäure-eicosylester.

Zur Herstellung des "Substanzgemisch" wird das Tensid oder eine Mischung von zwei Tensiden mit der 2,3 bis 100-fachen Gewichtsmenge - vorzugsweise mit der 24 bis 30-fachen Gewichtsmenge des Fettsäureesters oder Fettsäureestergemisches gemischt und die Mischung unter Rühren erwärmt, bis sie schmilzt. Die Schmelztemperatur ist naturgemäß von der Wahl der Komponenten und deren Mengenverhältnis abhängig und liegt normalerweise bei ca. 40° C bis 70° C.

Die Schmelze des "Substanzgemisch" wird dann unter Rühren in die 10 bis 200-fache Gewichtsmenge -vorzugsweise die 20 bis 120-fache Gewichtsmenge einer halbfesten Zubereitung eingetragen, die etwa auf die gleiche Temperatur (± ca. 5° C) erwärmt wurde, wie das "Substanzgemisches" selbst. Die hierzu verwendete halbfeste Zubereitung kann in konventioneller Weise als O/W-Emulsion oder Mischemulsion hergestellt werden und wird günstiger Weise als frisch zubereitete Formulierung zur Durchführung des erfindungsgemäßen Verfahrens verwendet. Geeignete Mischemulsionen sind beispielsweise die in der EP-A 0065929 beschriebene Doppelemulsion wenn diese verwendet werden; man hat in diesem Falle aber dafür Sorge zutragen, daß man niedrigschmelzende Freisetzungsgemische verwendet, da diese Doppelemulsionen bei Temperaturen oberhalb von 40° C teilweise entmischt werden können. Anschließend läßt man die halbfeste Zubereitung auf Raumtemperatur erkalten, versetzt sie mit dem auf maximal 80 µm mikronisierten Wirkstoff oder Wirkstoffgemisch sowie gewünschtenfalls noch zusätzlich mit Duftstoffen und rührt, bis diese Komponenten in der Zubereitung gleichmäßig verteilt sind.

Wirkstoffe, die sich zur Herstellung der erfindungsgemäßen halbfesten Zubereitungen eigenen, sind vorzugsweise solche, die bei Raumtemperatur in Wasser schwer löslich (maximal etwa 1 Gewichtsprozent) bis praktisch unlöslich sind. Vorzugsweise verwendet man sind solche Wirkstoffe, die auch konventioneller Weise zu Herstellung halbfester Zubereitungen für die topische Behandlung von Erkrankungen der Haut verwendet werden kann. Solche Wirkstoffe sind beispielsweise:
Antibiotika wie Erythromycin, Chloramphenicol, Tetracyclin oder Chlortetracyclin, Antiseptika wie Salicylsäure, Aknemittel wie Tretinoin, nichtsteroidale Antiphlogistika wie Crotaminton, Bufexamac, oder Indometacin und insbesondere Kortikoide wie Betamethason, Beclomethason, Chlocortolon, Difluorcortolon, Flumethason, Fluocinnolonacetamid, Fluocortinbutyl, Fluocortolon, Hydrocortison, 6α-Methylhydrocortison, 6α-Methylprednisolon, Prednicarbat, Triamcinolon, Triamcinolonacetamid und Ester dieser Kortikoide.

Es wurde bereits erwähnt, daß man die Wirkstoffe in mikronisierter Form von maximal 80 µm verwendet. Vorzugsweise haben die mikronisierten Wirkstoffe eine Korngröße von etwa 5 µm bis 40 µm. Die optimale Wirkstoffkonzentration ist unter anderem von der Art des verwendeten Wirkstoffs abhängig und muß im Einzelfall ermittelt werden.

Duftstoffe, die man gegebenenfalls in die erfindungsgemäßen halbfesten Zubereitungen einbauen kann, sind beispielsweise solche der Crematest®-Serie der Firma DRAGOCO.

Die nachfolgenden Ausführungsbeispiele dienen zur näheren Erläuterung der Erfindung.

### Beispiel 1

Eine Mischung von 3,5 g PEG 400-stearat, 7,5 g Rewomul MG® (Hersteller REWO Chem. Werke GmbH; DE-6497 Steinau 1), 2,0 g Cetylstearylalkohol, 6,5 g Octyldodecanol, 12,0 g weiße Vaseline und 4,0 g Mygliol® 812 (Hersteller Hüls AG) wird auf 70° C erhitzt. Dann versetzt man die entstandene Schmelze unter kräftigem Rühren mit einer auf 70° C erwärmten Mischung von 15,0 g wasserhaltigem Polyacrylat-Gel (DAB 9), 0,2 g Natriumcetylsulfat, 3,0 g Cera liquida, 5,0 g 85 %iges Glycerin, 1,0 g Benzylalkohol und 38,65 g bidestilliertem Wasser, läßt die Mischung auf 55° C erkalten und versetzt sie unter kräftigem Rühren mit einem 55° C warmen "Substanzgemisch" aus 0,1 g Pentaerythritmonooleat, 0,3 g PEG 400-stearat und 1,0 g Eicosensäureeicosenester. Man läßt die Emulsion unter Rühren auf Raumtemperatur erkalten und versetzt sie mit 0,25 g mikronisiertem 6α-Fluor-11β,21-dihydroxy-16α-methyl1,4-pregnadien-3,20-dion und rührt bis dieses homogen verteilt ist.

Die gebildete halbfeste Zubereitung zeigt am Patienten die gleiche Wirksamkeit wie eine 0,5 %iges 6α-Fluor-11β,21-dihydroxy-16α-methyl-1,4-pregnadien-3,20-dion Zubereitung in einer konventionellen Salbenzubereitung.

### Beispiel 2

Ein Gemisch von 10,0 g Cetylstearylalkohol, 25,0 g weiße Vaseline und 1,0 g Cera liquida wird auf 70° C erwärmt. Dann gibt man unter kräftigem Rühren eine 70° C warme Mischung von 5,0 g Polysorbat 60, 6,0 g 85 %igem Glycerin 1,0 g Benzylalkohol und 5,65 g bidestilliertem Wasser zu und läßt die halbfeste Zubereitung unter Rühren auf 50° C erkalten. Nun versetzt man die Mischung unter kräftigem Rühren mit einem 50° C warmen "Substanzgemisch" aus 0,1 g Pentaerythritmonooleat, 0,1 g Polysorbat 60 und 0,75 g Eicosensäuredocosenester, läßt sie auf Raumtemperatur abkühlen und verteilt in ihr homogen 0,5 g mikronisiertes D-(-)-treo-2-Dichlor-N(β-hydroxy-α-hydroxymethyl)-p-nitrophenylethyl)-acetamid.

### Beispiel 3

Eine Mischung aus 4,0 g Glycerinmonostearat, 4,0 g Cera liquida, 4,0 g Cetanol, 6,5 g Mygliol® 812 (Hersteller Hüls AG) und 25 g weiße Vaseline werden auf 70° C erhitzt und unter kräftigem Rühren mit einer 70° C warmen Mischung aus 7,0 g Polyethylenglycerinmonostearat, 2,9 g Propylenglykol, 1,0 g Benzylalkohol und 38,0 g Wasser versetzt. Man läßt die Mischung auf 52° C abkühlen und versetzt sie unter kräftigem Rühren mit einem 52° C warmen "Substanzgemisch" aus 0,1 g Pentaerythritmonostearat, 0,5 g PEG-2000-stearat und 4,0 g Docosensäureeicosenoid. Man läßt die halbfeste Zubereitung auf Raumtemperatur erkalten und verteilt in ihr homogen 3,0 g mikronisierte 1-(4-Chlorbenzoyl)-5-methoxy-2-methyl-indol-3-yl-essigsäure.

## Patentansprüche

1. Verfahren zur Herstellung von mikronisierte Wirkstoffe enthaltende halbfeste Zubereitungen,
dadurch gekennzeichnet, daß man ein Gemisch aus einem oder zwei nichtionischen Tensiden mit der 2,3 bis 100-fachen Gewichtsmenge eines Fertsäureesters der allgemeinen Formel
R₁ - COO - R₂
worin
R₁ und R₂ jeweils Alkylgruppen oder Alkenylgruppen mit 12 bis 32 Kohlenstoffatomen darstellen,
oder einem Gemisch dieser Fettsäureester erwärmt, bis es schmilzt,
die Schmelze unter Rühren in die 10 bis 200 - fache Gewichtsmenge einer halbfesten Zubereitung,
die etwa auf die gleiche Temperatur erwärmt ist, einträgt,
die Mischung unter Rühren erkalten läßt und
mit dem mikronisierten Wirkstoff oder Wirkstoffgemisch sowie gewünschtenfalls mit Duftstoffen versetzt,
wobei der mikronisierte Wirkstoff oder das Wirkstoffgemisch eine Korngröße von maximal 80 µm hat, und
wobei das Gemisch aus zwei Tensiden einen HLB-Wert von 9 bis 18 hat.

2. Verfahren zur Herstellung von mikronisierte Wirkstoffe enthaltende halbfeste Zubereitung gemäß Patentanspruch 1, dadurch gekennzeichnet, daß die halbfeste Zubereitung eine O/W - Emulsion oder eine Mischemulsion ist.

3. Verfahren zur Herstellung von mikronisierte Wirkstoffe enthaltende halbfeste Zubereitungen gemäß Patentanspruch 1 und 2, dadurch gekennzeichnet, daß die nichtionische Tenside in Wasser lösliche und in Paraffin unlösliche Oxethylate sind.

4. Verfahren zur Herstellung von mikronisierte Wirkstoffe enthaltende halbfeste Zubereitungen gemäß Patentanspruch 1 bis 3, dadurch gekennzeichnet, daß die Wirkstoffe ein Antibiotikum, ein nichtsteroidales Antiphlogistikum oder ein Kortikoid sind.

5. Mikronisierte Wirkstoffe enthaltende Zubereitung, dadurch gekennzeichnet, daß sie nach dem Verfahren gemäß Patentanspruch 1 bis 4 hergestellt sind.

## Claims

1. Method of preparing semi-solid preparations containing micronised active ingredients, characterised in that a mixture of one or two non-ionic surfactants with from 2.3 to 100 times the amount by weight of a fatty acid ester of the general formula
R₁-COO-R₂
wherein
R₁ and R₂ are each an alkyl group or alkenyl group having from 12 to 32 carbon atoms,
or a mixture of such fatty acid esters, is heated until it melts,
the melt is introduced, with stirring, into from 10 to 200 times the amount by weight of a semi-solid preparation which has been heated to approximately the same temperature,
the mixture is allowed to cool, with stirring, and
the micronised active ingredient or active ingredient mixture and, if desired, perfumes are added,
the micronised active ingredient or the active ingredient mixture having a particle size of at most 80 µm, and
the mixture of two surfactants having an HLB value of from 9 to 18.

2. Method of preparing semi-solid preparations containing micronised active ingredients according to patent claim 1, characterised in that the semi-solid preparation is an o/w emulsion or a mixed emulsion.

3. Method of preparing semi-solid preparations containing micronised active ingredients according to patent claims 1 and 2, characterised in that the non-ionic surfactants are water-soluble and paraffin-insoluble oxethylates.

4. Method of preparing semi-solid preparations containing micronised active ingredients according to patent claim 1 to 3, characterised in that the active ingredients are an antibiotic, a non-steroidal antiphlogistic or a corticoid.

5. Preparation containing micronised active ingredients, characterised in that it is prepared in accordance with the method according to patent claim 1 to 4.

## Revendications

1. Procédé de fabrication de préparations semi-solides contenant des principes actifs micronisés, caractérisé en ce que l'on incorpore à un mélange d'un ou deux agents de surface non ioniques une quantité 2,3 à 100 fois supérieure en poids d'un ester d'acide gras de la formule générale
R₁-COO-R₂
dans laquelle
R₁ et R₂ représentent l'un et l'autre des groupes alkyle ou des groupes alcényle possédant de 12 à 32 atomes de carbone, ou bien on chauffe un mélange de ces esters d'acides gras jusqu'à ce qu'il fonde, on introduit la matière fondue sous agitation dans une quantité 10 à 200 fois supérieure en poids d'une préparation semi-solide chauffée à une température à peu près égale, on laisse refroidir le mélange sous agitation et on le mélange avec le principe actif ou le mélange de principes actifs micronisés ainsi que, le cas échéant, avec des parfums, le principe actif ou le mélange de principes actifs micronisés présentant une granulométrie maximale de 80 µm et le mélange de deux agents de surface ayant un rapport HLB de 9 à 18.

2. Procédé de fabrication de préparations semi-solides contenant des principes actifs micronisés selon la revendication 1, caractérisé en ce que la préparation semi-solide est une émulsion huile-dans-eau ou une émulsion mixte.

3. Procédé de fabrication de préparations semi-solides contenant des principes actifs micronisés selon la revendication 1 et la revendication 2, caractérisé en ce que les agents de surface non ioniques sont des éthoxylates solubles dans l'eau et insolubles dans la paraffine.

4. Procédé de fabrication de préparations semi-solides contenant des principes actifs micronisés selon les revendications 1 à 3, caractérisé en ce que les principes actifs sont un antibiotique, un anti-inflammatoire non stéroïde ou un corticoïde.

5. Préparation semi-solide contenant des principes actifs micronisés, caractérisée en ce qu'elle est fabriquée suivant le procédé des revendications 1 à 4.
